# EUROPÄISCHE PATENTSCHRIFT

(11) **EP 1 003 477 B1**
(45) Veröffentlichungstag und Bekanntmachung des Hinweises auf die Patenterteilung: **09.04.2003**
(21) Anmeldenummer: 98939602.3
(22) Anmeldetag: 02.07.1998
(51) Int. Cl.: A61K 9/70

(54) **TRANSDERMALES THERAPEUTISCHES SYSTEM (TTS) ENTHALTEND AMMONIOGRUPPENHALTIGE (METH)ACRYLATPOLYMERE ZUR VERABREICHUNG VON LEVONORGESTREL**
THERAPEUTICAL SYSTEM FOR TRANSDERMAL DELIVERY OF LEVONORGESTREL
SYSTEME THERAPEUTIQUE D'ADMINISTRATION TRANSDERMIQUE DE LEVONORGESTREL

(30) Priorität: 04.07.1997 DE 19728516
(43) Veröffentlichungstag der Anmeldung: 31.05.2000
(73) Patentinhaber: SCHWARZ PHARMA AG, 40789 Monheim (DE)
(72) Erfinder: WOLFF, Hans-Michael, D-40789 Monheim (DE); ARTH, Christoph, D-40593 Düsseldorf (DE)
(86) Internationale Anmeldenummer: EP9804079
(87) Internationale Veröffentlichungsnummer: WO99001109

(56) Entgegenhaltungen:
- EP-A- 0 848 960
- DE-A- 3 823 070
- DE-A- 4 310 012
- CHAKKAPAN, S. ET AL: "Studies in transdermal drug delivery systems for estradiol" INDIAN J. PHARM. SCI. (1994), 56(4), 121-5 CODEN: IJSIDW;ISSN: 0250-474X, XP002087777
- KALBITZ J ET AL: "MODULATION DER WIRKSTOFFPENETRATION IN DIE HAUT" PHARMAZIE, Bd. 51, Nr. 9, 1. September 1996, Seiten 619-637, XP000621188

## Beschreibung

Die vorliegende Erfindung betrifft ein Transdermales Therapeutisches System (TTS) zur transepidermalen Verabreichung von Sexualsteroiden über längere Zeiträume, dadurch gekennzeichnet, daß das TTS eine steroidhormonhaltige Matrixmasse in Form einer Schicht aufweist, die mindestens 2 Gewichtsprozent Levonorgestrel oder mindestens 2 Gewichtsprozent Levonorgestrel und mindestens 2 Gewichtsprozent Estradiol, ammoniogruppenhaltige (Meth)acrylatcopolymere, mindestens einen Weichmacher aus der Gruppe der Ester schwacher organischer Säuren, ein Fettsäureester und Polyethylenglykol enthält.

Die Bioverfügbarkeit von oral verabreichten Wirkstoffen ist oft unbefriedigend. Die hepatische Metabolisierung vieler Wirkstoffe kann bei der ersten Leberpassage zu unerwünschten Konzentrationsverhältnissen, toxischen Nebenprodukten und zur Verminderung der Wirkung oder gar zum Wirkverlust führen. Gegenüber oraler Verabreichung besitzt die transdermale Gabe von Wirkstoffen verschiedene Vorteile. Die Wirkstoffzufuhr läßt sich über einen längeren Zeitraum besser steuern, wodurch hohe Blutspiegelschwankungen vermieden werden. Zudem kann die erforderliche therapeutisch wirksame Dosis meist deutlich verringert werden. Außerdem wird ein Pflaster vom Patienten oft mehr bevorzugt als täglich einmal oder mehrfach einzunehmende Tabletten.

In der Vergangenheit wurde zur Überwindung der vorgenannten Nachteile der nichttransdermalen Gabe von Wirkstoffen durch eine Vielzahl von Transdermalen Therapeutischen Systemen (TTS) mit unterschiedlichem Aufbau für verschiedene Wirkstoffe zur Therapie unterschiedlicher Erkrankungen Rechnung getragen.

So beschreiben die nachfolgend genannten technischen Dokumente für eine breite Vielfalt systemisch oder lokal reagierender Wirkstoffe deren parenterale Verabreichung entweder auf Basis dosis-kontrollierender oder allgemein freisetzender Systeme.

Beispielhaft sind dies: U.S.P.
3,598,122; 3,598,123; 3,731,683; 3,797,494; 4,031,894; 4,201,211; 4,286,592; 4,314,557; 4,379,454; 4,435,180; 4,559,222; 4,568,343; 4,573,995, 4,588,580; 4,645,502; 4,702,282; 4,788,062; 4,816,258; 4,849,226; 4,908,027; 4,943,435 und 5,004,610.

In den späten sechziger Jahren dieses Jahrhunderts war ursprünglich theoretisch angenommen worden, daß jeder Wirkstoff mit kurzer Halbwertszeit aber hoher Wirksamkeit und guter Hautdurchgängigkeit für eine sichere und effektive Verabreichung mittels eines TTS geeignet sei. Diese anfänglichen Erwartungen hinsichtlich der Möglichkeiten der transdermalen Verabreichung von Wirkstoffen mittels TTS konnten jedoch nicht erfüllt werden. Dies findet seine Begründung hauptsächlich darin, daß die Haut von Natur aus mit einer unüberschaubaren Vielfalt von Eigenschaften ausgestattet ist, um ihre Funktion als intakte Barriere gegenüber dem Eindringen von nicht-körpereigenen Substanzen in den Körper aufrecht zu erhalten. (Siehe hierzu: Transdermal Drug Delivery: Problems and Possibilities, B.M. Knepp et al., CRC Critical Review and Therapeutic Drug Carrier Systems, Vol. 4, Issue 1 (1987)).

Daher steht die transdermale Verabreichung nur für diejenigen wenigen Wirkstoffe zur Verfügung, die eine geeignete Kombination von vielen günstigen Charakteristika aufweisen. Für einen bestimmten Wirkstoff sind alle geforderten Charakteristika, die die sichere und effektive transdermale Verabreichung gewährleisten, weder theoretisch noch praktisch vorhersagbar.

Die an einen für die transdermale Verabreichung geeigneten Wirkstoff zu stellenden Anforderungen sind:
- Hautdurchgängigkeit,
- keine Beeinträchtigung des Klebevermögens des Pflasters durch den Wirkstoff,
- Vermeidung von Hautirritationen,
- Vermeidung von allergischen Reaktionen,
- günstige pharmakokinetische Eigenschaften,
- günstige pharmakodynamische Eigenschaften,
- ein relativ weites therapeutisches Fenster,
- Metabolismuseigenschaften, die konsistent mit der therapeutischen Anwendung bei kontinuierlicher Gabe sind.

Unzweifelhaft ist die vorgenannte Liste der Anforderungen nicht erschöpfend. Damit ein Wirkstoff für die transdermale Verabreichung zur Verfügung stehen kann, ist die "richtige" Kombination all dieser Anforderungen wünschenswert.

Das für die Wirkstoffe vorgenannte gilt in gleicher Weise für die den jeweiligen Wirkstoff enthaltende TTS-Zusammensetzung und deren konstruktiven Aufbau.

Üblicherweise handelt es sich bei den Transdermalen Therapeutischen Systemen (TTS) um Pflaster, die mit einer undurchlässigen Deckschicht, einer abziehbaren Schutzschicht und einer wirkstoffhaltigen Matrix oder einem wirkstoffhaltigen Reservoir mit semipermeabler Membran ausgestattet sind. Im ersten Fall werden sie als Matrixpflaster, im zweiten Fall als Membransystem bezeichnet.

Für die Deckschicht werden üblicherweise Folien aus Polyester, Polypropylen, Polyethylen, Polyurethan etc. verwendet, die auch metallisiert oder pigmentiert sein können. Für die abziehbare Schutzschicht kommen u.a. Folien aus Polyester. Polypropylen oder auch Papier mit Silikon- und/oder Polyethylenbeschichtung in Betracht.

Für die pharmazeutisch bzw. medizinisch üblichen wirkstoffhaltigen Matrices werden Polymermaterialien auf Basis von Polyacrylat, Silikon, Polyisobutylen, Butylkautschuk, Styrol/Butadien-Copolymerisat oder Styrol/Isopren-Copolymerisat verwendet.

Die in Membransystemen verwendeten Membranen können mikroporös oder semipermeabel sein und werden üblicherweise auf Basis eines inerten Polymeren, insbesondere Polypropylen. Polyvinylacetat oder Silikon gebildet.

Während die wirkstoffhaltigen Matrixzusammensetzungen selbstklebend sein können, ergeben sich aber auch in Abhängigkeit von eingesetztem Wirkstoff wirkstoffhaltige Matrices, die nicht selbstklebend sind, so daß als Folge hiervon das Pflaster oder TTS konstruktiv mit einem Overtape versehen werden muß.

Zur Sicherstellung der erforderlichen Fluxrate des Wirkstoffes sind häufig Hautpenetrationsenhancer wie aliphatische, cycloaliphatische und/oder aromatisch-aliphatische Alkohole, jeweils ein- oder mehrwertig und jeweils mit bis zu 8 C-Atomen umfassend, ein Alkohol/Wasser-Gemisch, ein gesättigter und/oder ungesättigter Fettalkohol mit jeweils 8 bis 18 Kohlenstoffatomen, eine gesättigte und/oder ungesättigte Fettsäure mit jeweils 8 bis 18 Kohlenstoffatomen und/oder deren Ester sowie Vitamine als Zusatz erforderlich.

Weiterhin werden häufig Stabilisatoren. wie Polyvinylpyrrolidon, α-Tocopherolsuccinat, Propylgallat, Methionin, Cystein und/oder Cystein-hydrochlorid, der wirkstoffhaltigen Matrix zugesetzt.

Wie die vorgenannte Aufstellung zeigt, sind zahlreiche TTS-Konstruktionen und hierfür verwendete Materialien bekannt. Allerdings sind viele interagierende Erfordernisse zu berücksichtigen, wenn ein Medikament in Form eines TTS einem medizinischen Bedürfnis genügen soll.

Die folgenden Problemstellungen sind bei der Entwicklung von wirkstoffhaltigen TTS grundsätzlich zu berücksichtigen:
1. Zum Erreichen therapeutisch notwendiger Penetrationsraten des Wirkstoffes durch die Haut ist meist eine hohe Wirkstoffbeladung der Polymermatrix erforderlich. Nach Applikationsende im TTS verbleibender Wirkstoff wird therapeutisch ungenutzt mit dem Pflaster entsorgt. Dies ist jedoch, insbesondere bei hochwirksamen und teuren Wirkstoffen, aus Umweltschutz- und Kostengründen unerwünscht.
2. Die wirkstoffbeladene und ggf. zusätzlich mit Hautpenetrationsenhancern beladene Polymermatrix ist bei längerer Lagerung physikalisch nicht stabil. Insbesondere kann eine Wirkstoffrekristallisation auftreten, die zu einer nicht kontrollierbaren Abnahme der Wirkstofffreisetzungskapazität des TTS führt.
3. Eine hohe Beladung des polymeren Trägerstoffes mit Wirkstoff und/oder Hautpenetrationsenhancern erschwert bei selbstklebenden Polymerfilmen die Einstellung optimaler Hafteigenschaften des transdermalen Systems.
4. Die Wirkstoffresorptionsrate sinkt bei Anwendungen über mehrere Tage in nicht akzeptabler Weise ab, so daß zusätzliche Steuerschichten und/oder -komponenten erforderlich sind.
5. Werden wirkstoffbeladene Schichten aus organischen Lösungen hergestellt, tritt das Problem des Verbleibens von Lösemittelresten in der wirkstoffhaltigen Schicht nach dem Trocknungsprozeß auf. Zusätzlich besteht die Gefahr einer unerwünschten Verdunstung von flüchtigen Hilfsstoffen während der Herstellung. Da aus Gründen der physikalischen Stabilität und Hautverträglichkeit des Systems in der Regel vollständige Lösungsmittelfreiheit anzustreben ist, muß das Reservoir gegebenenfalls in mehreren Schichten aufgebaut werden. Dies wiederum führt zu einer Erhöhung der Herstellungskosten.

Die beschriebenen Probleme bedingen daher eine Vielzahl von Ausführungsformen Transdermaler Therapeutischer Systeme, die sich im Stand der Technik auf diesem Gebiet widerspiegeln.

Eine neuere Übersicht hierzu gibt beispielsweise **U.S. P 5,662,926** (Wick et al., 1997). Dieses Dokument beschreibt transdermale Systeme, die einen monolithischen, thermoplastischen Polymerfilm enthalten, in dem ein Wirkstoff, vorzugsweise Nikotin, homogen verteilt ist, sowie ein Verfahren zur lösungsmittelfreien Herstellung dieser wirkstoffhaltigen Schicht durch Mischen des wirksamen Bestandteils mit dem polymeren Trägermaterial in der Polymerschmelze bei Temperaturen von 170°C bis 200°C. Zur Fixierung des wirkstoffhaltigen Matrixfilms auf der Haut dient ein zusätzlicher Kontaktkleberfilm, der auf die Wirkstoffmatrix aufgebracht wird und, falls erforderlich. zusätzlich ein flächengrößeres Pflaster, das auf der von der Haut abgewandten Matrixseite auf den wirkstoffhaltigen Polymerfilm aufgebracht wird.

Besondere pharmazeutisch technische Probleme sind bei der Entwicklung von Östrogenpflastern zu lösen, die zur Behandlung klimakterischer Beschwerden appliziert werden müssen. Die Applikation sollte nur ein- oder zweimal wöchentlich erfolgen. Zunehmend Beachtung finden dabei sog. 7-Tage-Pflaster aus Kosten- und Patientencompliancegründen in dieser Indikation. Kostenaspekte spielen hier insofern eine besondere Bedeutung, als viele Sexualsteroidhormone hochpreisige Arzneistoffe darstellen, die für eine Dauertherapie vorgesehen sind. Zudem ist bei der Hormonverabreichung aus medizinischen Gründen nicht selten eine Kombinationstherapie erwünscht. So wird das natürliche Östrogen - 17β-Estradiol - zur Behandlung von klimakterischen Beschwerden in der Regel entweder kontinuierlich oder sequentiell zusammen mit einem Gestagen verabreicht.

Eine hinlänglich bekannte Ausführungsform solcher TTS stellen monolithische Wirkstoffpflaster dar, die eine kontrollierte Abgabe der wirksamen Bestandteile aus einer dünnen Haftklebeschicht ermöglichen. In der Praxis stößt jedoch die Entwicklung solcher Wirkstoffpflaster mit Sexualsteroidhormonen, insbesondere bei einer Anwendung über mehrere Tage, auf eine oder mehrere der nachfolgend genannten Schwierigkeiten, die häufig nur durch aufwendige Maßnahmen umgangen werden können und die Entwicklungs- und/oder Herstellkosten erhöhen. Es handelt sich im wesentlichen um folgende Problemstellungen:
1. Das Sexualsteroidhormon wird aus den Haftkleberfilmen in einer relativ niedrigen Rate pro Zeiteinheit durch die Haut freigesetzt. mit der Folge, daß relativ großflächige Pflaster appliziert werden müssen, um therapeutisch notwendige Hormonspiegel im Blut über längere Zeit aufzubauen und/oder daß sog. Penetrationsbeschleuniger zusammen mit dem oder den wirksamen Bestandteilen verabreicht werden müssen, um die erforderliche transepidermale Transportrate zu erzielen.
2. Das Sexualsteroidhormon ist in dem selbstklebenden Film in Abhängigkeit von den Aufbewahrungsbedingungen physikalisch instabil, d.h. es besteht insbesondere die Gefahr einer Wirkstoffrekristallisation bei Lagerung, die verbunden ist mit einer nicht kontrollierbaren Abnahme der Wirkstofffreisetzungskapazität.
3. Die Wirkstoffresorptionsrate sinkt bei Anwendung über mehrere Tage in nicht akzeptabler Weise, so daß zusätzliche Steuerschichten oder -komponenten erforderlich sind.
4 Eine hohe Beladung mit Wirkstoff und Penetrationsbeschleunigern erschwert in der Entwicklung die Einstellung optimaler Hafteigenschaften des TTS.
   Besondere Probleme stellen z.B. ein kalter Fluß der selbstklebenden Reservoirschicht dar, der bei der Humananwendung zu einem Austreten von wirkstoffhaltiger Masse über den Pflasterrand hinaus und damit zu Schmutzrändern führen kann. Weiterhin ist teilweises oder vollständiges Ablösen des TTS, bedingt durch Feuchtigkeitseinwirkung (z.B. beim Duschen, Schwimmen, starkem Schwitzen) und/oder durch starke Scherbeanspruchungen infolge von Muskel- resp. Hautbewegungen an der Grenzfläche Haut/Pflaster festzustellen.
5. Reservoirschichten für die transdermale Applikation werden häufig aus Lösungen hergestellt, so daß das Problem des Verbleibens von Lösemittelresten in der wirkstoffhaltigen Schicht nach dem Trocknungsprozeß und gegebenenfalls der damit erfolgenden Abdampfung und/oder unerwünschten Verdunstung von flüchtigen Hilfsstoffen während der Herstellung auftritt. Um vollständige Lösungsmittelfreiheit zu erzielen, die aus Gründen der physikalischen Stabilität und der Hautverträglichkeit des Systems in der Regel anzustreben ist, muß das Reservoir gegebenenfalls in mehreren Schichten aufgebaut werden, was jedoch zu einer Verteuerung der Herstellung führt.

Zur transdermalen Anwendung von Östrogenen und/oder Gestagenen und/oder Androgenen mittels monolithischer Systeme, bei denen der oder die Wirkstoffe in eine selbstklebende Haftklebermatrix eingearbeitet werden, kommen nach dem Stand der Technik - u.a. wegen ihres relativ guten Lösungsvermögens für diese Wirkstoffgruppe - bevorzugt Haftkleber auf Acrylat-Copolymerisatbasis zum Einsatz, ohne (EP 0 416 8412, WO 93/10772) oder mit (WO 96/08255, DE 44 05 898) Zusatz von penetrationsfördernden, kristallisationshemmenden (WO 95/09618, WO 93/08795), die Wirkstofflöslichkeit weiter erhöhenden (DE OS 44 05 898) und wasserbindenden (DE 39 33 460) Substanzen.

Die beschriebenen Rezepturen erfordern dabei in der Regel den Einsatz organischer Lösungsmittel, die im Rahmen der Herstellung wieder quantitativ entfernt werden müssen. Auch sind - trotz des relativ einfachen Aufbaus monolithischer TTS - die üblichen pharmazeutischen Qualitätsanforderungen bzgl. Hafteigenschaften, Reproduzierbarkeit der Wirkstofffreisetzung und Lagerstabilität wegen der oben beschriebenen Schwierigkeiten nur mit hohem technischen Aufwand in der Entwicklung und Produktion zu gewährleisten. Häufig müssen großflächige Pflaster appliziert werden, insbesondere zur Verabreichung von Gestagenen, um die therapeutisch erforderlichen Wirkstoffspiegel im Blut über eine mehrtägige Anwendungsdauer aufrechtzuerhalten, wodurch sich zum einen die Gebrauchseigenschaften und damit verbunden die Patientencompliance verschlechtern, zum anderen die Kosten für das Präparat weiter erhöhen.

Ferner sind aus der Literatur monolithische Systeme mit Sexualsteroiden auf Basis von Polystyrolblockcopolymeren als Trägermaterialien bekannt, deren Verwendung prinzipiell eine Fertigung selbstklebender Wirkstoffreservoire aus der Schmelze ohne Einsatz von organischem Lösungsmittel erlaubt. So werden in WO 94/26257 steroidhaltige Haftkleber beschrieben, die Ester des Kolophoniums enthalten und bei denen die Herstellung einer estradiol- und/oder levonorgestrelhaltigen Klebermatrix durch Aufschmelzen und intensives Kneten bei hoher Temperatur über lägere Zeiträume erfolgen kann. Transdermale Therapeutische Systeme, die auf diese Weise hergestellt werden, weisen den Nachteil auf, daß sich der oder die Wirkstoffe und/oder pharmazeutische Zusätze unter den Bedingungen des Herstellprozesses teilweise zersetzen, daß die Hafteigenschaften und/oder Hautverträglichkeit des Pflasters über mehrere Tage unzureichend sind, und - insbesondere für die Gestagenkomponente - die erzielbaren Plasmakonzentrationen therapeutisch nicht ausreichen.

Aus der EP 0 186 019 sind ferner Wirkstoffpflaster bekannt, bei denen einer Kautschukklebeharzmasse in Wasser quellbare Polymere zugesetzt sind und aus denen Estradiol freigesetzt werden kann, wobei im Einzelfall ebenfalls eine Herstellung nach dem Hotmelt-Verfahren möglich ist. Auch bei diesen Rezepturen ist es schwierig, ausreichende Mengen an Sexualsteroidhormonen in der Pflastermatrix in Lösung zu halten und über längere Zeiträume in einer annähernd konstanten Rate durch die Haut freizusetzen.

Femer sind aus der DE 44 29 667 Rezepturen für die transepidermale Abgabe von Estradiol bekannt, die ohne Verwendung von organischen Lösungsmitteln durch Aufschmelzen der Rezepturbestandteile hergestellt werden, wobei als Schutz vor Ausfällung des Estradiol-Hemihydrates bei Lagerung ein Zusatz von Glycerin vorgesehen ist. Die in der Beschreibung bzw. in den Beispielen genannten Kleberformulierungen auf Basis von Polystyrolblockcopolymeren entsprechen dabei dem Stand der Technik, d.h. Wirkstoffaufnahme und -abgabekapazität von TTS dieser Art sind für eine Anwendung des Hormonpflasters über mehrere Tage im allgemeinen, insbesondere im Fall von Gestagenen und Androgenen, zu niedrig.

Aus dem Indian J. Pharm. Sci (1994), 56(4), 121-5 ist ein transdermales therapeutisches System bekannt, enthaltend Estradiol als Wirkstoff, ammoniogruppenhaltiges (Meth)-acrylatcopolymer (Eudragit RL), Triacetin und Zitronensäure, im Gegensatz zu Estradiol ist Levonorgestrel ein Gestagen, das keine phenolische Hydroxylgruppe enthält und daher gegenüber Estradiol völlig verschiedene physikochemische Eigenschaften hat. Damit einhergehend unterscheiden sich Estradiol und Levonorgestrel deutlich in ihrer Löslichkeit in TTS-Grundlagen sowie ihrer Fähigkeit zur Hautdurchdringung.

Auch erfolgt die Herstellung nach dem Lösungsmittelverfahren, so daß während derselben zugegebenes Lösungsmittel wieder entfernt werden muß.

DE 38 23 070 A1 offenbart ein transdermales therapeutisches System in Form einer Wirkstoffmatrix mit einem aminogruppenhaltigen (Meth)acrylatcopolymeren, einer klebrigmachenden Substanz, einem Fettsäureester als Resorptionsvermittler sowie Estradiol als Wirkstoff. Im Hinblick auf den Wirkstoff sowie die Herstellung des Pflasters gilt das zu vorgenanntem Literaturzitat Ausgeführte. Auch ist neben dem matrixbildenden Trägerpolymer der Zusatz einer klebrigmachenden Substanz erforderlich, um die Anwendbarkeit des Pflasters durch Aufkleben auf die Haut zu gewährleisten.

Neben den monolithischen Systemen sind aus der Literatur auch mehrschichtig aufgebaute Matrix- und Reservoirsysteme hinlänglich bekannt, bei denen Wirkstoffreservoir-, Haftklebeschicht und/oder Freisetzungskontrollschichten funktionell und/oder räumlich voneinander getrennt sind. Die EP 0 285 563 beschreibt ein TTS für die kombinierte Applikation von Östrogenen und Gestagenen. Das Wirkstoffreservoir enthält dabei Ethanol als Lösungs- und Freisetzungssteuerungsmittel für die wirksamen Bestandteile. An der Kontrolle der Steroidhormonfreisetzung ist ferner eine Membran beteiligt, die sich zwischen Reservoir und separat angeordneter Klebeschicht befindet. Die mögliche Anwendungsdauer solcher TTS hängt u.a. stark vom Ethanolgehalt im Reservoir ab (J.A.

Simon et al. (1991), Fertility and Sterility, 56: 1029-1033), der bei der Humananwendung durch Resorption fortlaufend abnimmt und damit die funktionale Lebenszeit des Systems begrenzt. Da neben den Wirkstoffen eine weitere, die Resorption steigernde Komponente in einer relativ hohen Rate freigesetzt wird, besteht je nach Umgebungs-, Lagerungs- und Anwendungsbedingungen das Risiko physikalischer Instabilität, nachlassender Klebkraft und/oder lokaler Hautirritationen.

Ein sog. "enhanced" System, bei dem außer dem Wirkstoff noch Penetrationsbeschleuniger an die Haut freigesetzt werden und welches getrennte Reservoir-, Steuerungs- und Klebeschichten enthält, ist aus dem Stand der Technik ebenfalls für die transepidermale Anwendung von Testosteron (U.S. 5,152,997) bekannt.

Dieses TTS hat für den Patienten den Vorteil, daß es nicht auf die relativ durchlässige Skrotalhaut geklebt werden muß, was wegen sonst zu geringer Wirkstoffabsorption bei Testosteronpflastern ohne Penetrationsvermittler (z.B. gemäß DE OS 35 23 065) der Fall ist. Eine Anwendung solcher "enhanced" Systeme über mehr als 24 Stunden ist jedoch mit einem erhöhten Risiko lokaler Hautreizungen verbunden, bedingt durch die die Hautpermeation von Testosteron steuernden Zusatzstoffe. Es können insbesondere bei ungünstigen Applikationsbedingungen (Schwitzen, starke Hautbewegungen, Duschen) Probleme bzgl. der Hafteigenschaften auftreten.

Schließlich sind in der Entwicklung von transdermalen Systemen Polymere auf Acrylsäureester und Methacrylsäureesterbasis von besonderem Interesse wegen ihres relativ guten Aufnahme- und Abgabevermögens für eine Vielzahl von Wirkstoffen. Um die Verwendung von Lösungsmitteln bei der Herstellung von Matrixsystemen auf Poly(meth)acrylatbasis zu vermeiden, ist in **DE 4310012** ein dermales therapeutisches System beschrieben, in dem eine oder mehrere Schichten aus Mischungen von Poly(meth)acrylaten aufgebaut und aus der Schmelze hergestellt werden und die erste Mischungskomponente aus (Meth)acrylpolymeren besteht, die funktionelle Gruppen enthalten, die zweite Mischungskomponente das Fließverhalten reguliert und nur unerhebliche Mengen an funktionellen Gruppen enthält. Die zusammengesetzten Systeme mit Poly(meth)acrylaten mit funktionellen Gruppen sollen eine gesteuerte Abgabe des oder der Wirkstoffe an bzw. durch die Haut und eine einfache Art der Herstellung ermöglichen. Den Vorteilen in der Herstellung gegenüber lösungsmittelbasierten Verfahren stehen bei solchen Systemen jedoch erfahrungsgemäß eine Reihe von Nachteilen gegenüber, die bedingt sind durch:
1. Länger andauernde thermische Belastung aller TTS-Komponenten bei (1) der Herstellung der Polymerschmelze, (2) der homogenen Einarbeitung des oder der Wirkstoffe und/oder (3) der Beschichtung der heißen wirkstoffhaltigen Masse auf geeignete Trägermaterialien mit erhöhtem Risiko von Abbau- bzw. Zersetzungsreaktionen in der Polymerschmelze und/oder während der Lagerung der wirkstoffhaltigen Polymerfilme.
2. Schwierigkeiten in der Optimierung der Co-/Adhäsionsbalance der poly(meth)acrylathaltigen Schicht, da eine Vernetzung des Acrylatcopolymerisates mittels kovalenter Bindungen während der Herstellung der wirkstoffhaltigen Polymermatrix in der Schmelze nicht möglich ist, verbunden mit Problemen, die durch einen kalten Fluß der Polymermasse bei Anwendung auf der Haut und/oder bei Lagerung auftreten können.
3. Starke Bindung der Wirkstoffe/Steroidhormone, insbesondere von 17-β-Estradiol in der Polymermatrix durch Polymethacrylate mit einem hohen Gehalt an freien Aminogruppen, wodurch die Fluxraten des Sexual-steroids gegenüber Polymethacrylat-Matrices ohne freie Aminogruppen - bei gleicher Wirkstoffbeladung - verringert werden (siehe Abb. 1, Vergleichsbeispiel).

Wie die vorgenannte Aufstellung zeigt, sind viele Pflasterkonstruktionen und hierfür verwendete Materialien bekannt. Gleichwohl besteht bis heute für viele in Transdermalen Therapeutischen Systemen verarbeitete Wirkstoffe ein großer Bedarf, TTS zur Verfügung zu stellen, die eine therapeutisch geforderte Wirkstoffabgabe ermöglichen, ohne dabei konstruktiv aufwendig zu sein und in der Gesamtschau ihrer Bestandteile eine optimale Beziehung darstellen.

Dies gilt auch für den Wirkstoff Levonorgestrel, wenn er transcutan verabreicht werden soll.

Aufgabe der vorliegenden Erfindung ist es daher, die vorgenannten Nachteile der TTS mit Sexualsteroiden zu vermeiden und ein konstruktiv einfaches, hautverträgliches, über längere Lagerungs- und Applikationsdauer physikalisch und chemisch stabiles TTS zur transepidermalen Verabreichung von Levonorgestrel, allein oder mit Estradiol mit guten Hafteigenschaften zur Verfügung zu stellen, das
a) pro Flächeneinheit möglichst viel Wirkstoff an und durch die Haut freisetzt,
b) lösungsmittelfrei ist und
c) bei dem der eingesetzte Wirkstoff bzw. die Wirkstoffe thermisch minimal belastet ist bzw. sind.

Zur Lösung dieser Aufgabe wird ein TTS, den Wirkstoff Levonorgestrel allein oder mit Estradiol enthaltend und ein Verfahren zu seiner Herstellung ohne Verwendung von Lösemitteln zur Verfügung gestellt, dessen besondere Zusammensetzung, entsprechend den Patentansprüchen, überraschenderweise der vorgenannten Aufgabenstellung genügt.

Das erfindungsgemäße Transdermale Therapeutische System (TTS) enthält eine Levonorgestrel enthaltende Matrixmasse in Form einer Schicht, wobei die Matrixmasse bei bis zu 200°C schmelzextrudierte, ammoniogruppenhaltige (Meth)acrylatcopolymere und mindestens einen Weichmacher und einen Fettsäureester und Polyethylenglykol sowie wenigstens 2 Gew.-% von jedem in der Matrixmasse vorhandenen nicht vorgeschmolzen eingearbeiteten Steroidhormon aufweist und nach außen mit einer Deckschicht versehen ist. Das erfindungsgemäße TTS bedarf keiner weiteren Klebeschicht zur Fixierung auf der Haut. Die Matrixschicht ist sowohl wirkstoffhaltig als auch das TTS hautfixierend. Die aus den erfindungsgemäßen TTS erzielbaren Freisetzungsraten sind so hoch, daß die Applikationszeit gegenüber den aus dem Stand der Technik bekannten Pflastersystemen verlängert werden kann, ohne die Applikationsfläche zu vergrößern (vergl. Abb. 1).

In der Entwicklung des erfindungsgemäßen TTS ist es überraschend gelungen, vorteilhafterweise Ko-/Adhäsions-eigenschaften des TTS einerseits und Wirkstofflöslichkeit, -lösungsgeschwindigkeit und -freisetzungsverhalten andererseits zu optimieren. Besonders überraschend ist die erhöhte Freisetzung des Wirkstoffes Levonorgestrel aufgrund der erfindungsgemäßen Kombination von ammoniogruppenhaltigen (Meth)arylatcopolymeren mit Triethylcitrat und dem Wirkstoff Levonorgestrel. Ferner ist für die erfindungsgemäßen TTS überraschend, daß
(1) gegenüber dem Stand der Technik hohe Wirkstoffkonzentrationen in der Polymermatrix gleichzeitig eine ausreichende physikalische Stabilität des Systems bei Langzeitlagerung gewährleistet und daß
(2) auf die Einbringung von separaten Trennschichten oder Membranen zwischen wirkstoffhaltiger und -freier Schicht verzichtet werden kann.

Überraschenderweise ergänzen sich in dem gattungsgemäßen TTS der vorliegenden Erfindung die hervorragenden Fluxeigenschaften der wirkstoffhaltigen Matrix mit ihrer hervorragenden Hafteigenschaft. Unmittelbar nach dem Aufkleben des TTS wird ein inniger Kontakt zwischen Wirkstoffmatrix und Haut hergestellt. Werden nach einer Ausführungsform der vorliegender Erfindung vernetzbare Haftkleberschichten als Fixierungshilfe direkt auf die Wirkstoffschicht kaschiert, weisen die so erhaltenen selbstklebenden TTS bestehend aus Deckschicht, Wirkstoffschicht und Haftschicht eine über längere Applikationszeiträume ebenfalls überraschend hohe Steroidfreisetzungsrate auf.

Nach einer weiteren Ausführungsform der Erfindung kann das aus Deckschicht und Wirkstoffschicht bestehende TTS mit Ausnahme seiner Freisetzungsfläche auf der Haut von einem größeren wirkstofffreien Hautpflaster zur Fixierung an der Applikationsstelle umgeben sein. Bei dieser Ausführungsform kann diese Fixierungshilfe auf Grund der hervorragenden Klebeeigenschaften der wirkstoffhaltigen Matrixschicht, ihren Überstand betreffend, sehr schmal sein. Vorteilhafterweise umgibt diese Fixierungshilfe das TTS jeweils randseitig mit 2-4 mm.

Besonders erfindungsgemäß vorteilhaft ist, daß das TTS den jeweiligen Wirkstoff als thermisch minimiert belastet enthält. Er wird ungeschmolzen der bei der Schmelzextrusion erhitzten Matrixmasse zugeführt.

Vorteilhaft ist die erfindungsgemäße Ausführungsform, in der die steroidhormonhaltige Matrixmasse eine feste Lösung ist.

Die erfindungsgemäße steroidhormonhaltige Matrixmasse enthält als Weichmacher Zitronensäuretriester sowie bevorzugt als Fettsäureester einen Ester der Ölsäure oder Nonansäure.

Ausführungsformen der Erfindung umfassen TTS, die Östrogene oder Gestagene alleine oder in Kombination enthalten.

Vorteilhafterweise weist die für das TTS verwendete Trägerfolie matrixseitig eine Metalldampf- oder Oxidbeschichtung auf.

Im Sinne der Erfindung werden die nachfolgend genannten Begriffe und/oder Worte, wie folgt, verstanden:
- a) "lösemittelfrei":: zur Herstellung der Polymermatrices werden keine Lösungsmittel verwendet, die im Verlaufe des Herstellungsverfahrens wieder weitgehend entfernt werden, wie dieses im sogenannten "solvent based"-Verfahren geschieht.
- b) "längere Appli-:: die TTS können zur therapeutischen Anwendung bis zu 7 Tage kationszeit- auf die Haut appliziert werden. räume":
- c) "feste Lösung":: der pharmazeutische Wirkstoff liegt in der Pflastermatrix molekulardispers verteilt vor.
- d)"transepi-dermal":: ist sinn- und funktionsgleich mit transcutan
- e) "thermisch minimiert belasteter Wirkstoff":: der Wirkstoff wird ungeschmolzen der bei der Schmelzextrusion erhitzten Matrixmasse zugeführt, die nach Wirkstoffzusatz gegekühlt wird.

Das Verfahren zur Herstellung des erfindungsgemäßen TTS ist dadurch gekennzeichnet, daß eine beschichtungsfähige steroidhormonhaltige Matrixmasse durch Schmelzextrusion erzeugt wird, wobei die wirksamen Bestandteile in eine bis zu 200°C heiße Polymerschmelze kontinuierlich eingewogen und nicht vorgeschmolzen eingearbeitet werden, die heiße wirkstoffhaltige Polymerschmelze direkt anschließend auf eine ablösbare Schutzschicht (= Substrat) in einer Dicke von 0,02 bis 0,4 mm beschichtet wird und das erhaltene 2-Schichtlaminat mit einer Deckschicht versehen wird.

Falls gewünscht, kann auch ein wirkstofffreier Klebefilm aus einem vernetzten Acrylatcopolymeren direkt auf die wirkstoffhaltige Polymermatrix laminiert werden. Die erfindungsgemäßen TTS werden mit einer Schutzfolie versehen, die vor Aufbringen des Präparates auf der Haut entfernt wird.

Ein wesentlicher Vorteil des erfindungsgemäßen Verfahrens besteht darin, daß das Wirkstoffreservoir (I) ohne Verwendung von organischen Lösungsmitteln hergestellt wird, und (II) die Zubereitung der wirkstoffhaltigen Matrixmasse und ihre weitere Verarbeitung zu einer wirkstoffhaltigen Schicht in einem kontinuierlichen und kosteneinsparenden Arbeitsgang erfolgen: Prozeßzeiten lassen sich auf wenige Minuten verkürzen und damit zugleich die Gefahr von Zersetzungsreaktionen in der wirkstoffhaltigen Polymerschmelze auf ein Minimum reduzieren. Überraschend wurde dabei gefunden, daß die vollständige Auflösung des oder der Sexualsteroide in der Polymerschmelze trotz der kurzen Prozeßzeiten unter den in den Beispielen weiter erläuterten Verfahrensbedingungen gewährleistet ist.

Ferner werden durch die kontinuierliche Herstellung der steroidhormonhaltigen Polymermasse Scaling-Up Probleme umgangen. D.h. bei Erhöhung des Ansatz- bzw. Chargengröße ist zur Herstellung der wirkstoffhaltigen Polymerschmelze und des Laminates kein Wechsel aus größere Produktionsanlagen erforderlich, der überlicherweise mit zeit- und kostenaufwendigen Installations-, Qualifizierungs- und Validierungsarbeiten sowie ggf. auch Rezepturänderungen verbunden ist.

Die Erfindung wird anhand folgender Beispiele erläutert:
Kombinations-TTS mit Levonorgestrel und Estradiol

### I. Beispiele 1 bis 4

Ein mit zwei Dosierern ausgerüsteter gleichsinnig laufender Zweischneckenextruder wird kontinuierlich in zwei aufeinanderfolgenden Verfahrenszonen mit einer homogenen Feststoffmischung A sowie einer flüssigen Mischung B beschickt. (Zur Zusammensetzung der Mischungen A und B siehe Tabelle 1.) Der Ansatz wird mit einem Gesamtdurchsatz von 1 kg/h bei einer Temperatur von 150°C schmelzextrudiert, wobei aus Dosierer 1 Mischung A in das erste Verfahrensteil in einer Rate von 690 g/h und aus Flüssigdosierer 2 Mischung B in das zweite Verfahrensteil in einer Rate von 310 g/h eingewogen wird.

Die erhaltene heiße Polymerschmelze wird auf eine ca. 100 µm dicke silikonisierte Polyesterfolie (= Schutzfolie) als Schicht aufgetragen, daß das Auftragsgewicht der Polymermasse ca. 80 g pro m² beträgt. Das Zweischichtmaterial, bestehend aus Schutzfolie und Matrixmasse, wird nach Abkühlen auf Raumtemperatur mit einer ca. 20 µm dicken Polyesterfolie (= Trägerfolie) abgedeckt und das so erhaltene Dreischichtlaminat in 16 cm² große Stücke ausgestanzt.

Die resultierenden Kombinations-TTS enthalten die in Tabelle 2 angegebenen Wirkstoffgehalte.

Die Vergleichsbeispiele 1 und 4 in Tabelle 1 dienen zur Unterscheidung zu den erfindungsgemäßen in den Beispielen 2 und 3 genannten TTS.

**Tabelle 1**

| Zusammensetzung der TTS (Beispiele 1 bis 4) | | | | |
|---|---|---|---|---|
| Bestandteil | Vergleichsbeispiel 1 Gehalt in Gew.-% | Beispiel 2 Gehalt in Gew.-% | Beispiel 3 Gehalt in Gew.-% | Vergleichsbeispiel 4 Gehalt in Gew.-% |
| **Mischung A** | | | | |
| Levonorgestrel | 2,9 | 7,0 | 7,0 | 2,9 |
| Estradiol | 4,3 | 3,6 | 3,6 | ./. |
| Eudragit RL 100 | 30,9 | ./. | ./. | ./. |
| Eudragit RS 100 | ./. | 44,7 | 44,7 | 97,1 |
| Eudragit E 100 | 61,9 | 44,7 | 44,7 | ./. |
| **Mischung B** | | | | |
| Triethylcitrat | 67,7 | ./. | ./. | ./. |
| Tributycitrat | ./. | 51,0 | 51,0 | 100,0 |
| Polyethylenglykol 400 | 32,3 | 32,6 | 32,6 | ./. |
| Ölsäureethylester | ./. | 16,4 | ./. | ./. |
| Nonansäureethylester | ./. | ./. | 16,4 | ./. |

### II. In vitro-Untersuchungen

### Hautpermeationsmessungen in vitro

Zur Beurteilung der Wirkstofffreisetzung in vitro wird ein TTS mit einer ausgestanzten Fläche von 5 cm² in einer modifizierten Franz-Diffusionszelle auf einer Hautpräparation haarloser Mäuse befestigt. Unmittelbar anschließend wird die Zelle mit destilliertem Wasser als Freisetzungsmedium luftblasenfrei befüllt und auf 32 ± 0,5°C thermostatisiert.

Zu den Probeentnahmezeiten wird das Freisetzungsmedium gegen frisches, auf 32 ± 0,5°C thermostatisiertes Wasser ausgetauscht.

Der Wirkstoffgehalt in dem jeweils entnommenen Freisetzungsmedium wird mittels Hochdruckflüssigkeitschromatographie bestimmt. Die Ergebnisse der Untersuchungen sind in Tabelle 2 für Beispiele 1 bis 4 und einem handelsüblichen Matrixkombinationspflaster mit einer deklarierten Freisetzung von 50 µg Estradiol und 20 µg Levonorgestrel pro Tag dargestellt. Wie die Gegenüberstellung der Fluxraten in Tabelle 2 zeigt, wird aus den erfindungsgemäßen TTS deutlich mehr Levonorgestrel durch die Haut freigesetzt als beim Referenzpflaster. Selbst bei Beispiel 4, das keinen Penetrationsvermittler enthält und gegenüber den Beispielen 1 bis 3 eine geringere Wirkstoffbeladung aufweist, liegt die Fluxrate nach 24 Std. vergleichsweise noch höher als bei dem Handelspräparat, das einen Penetrationsvermittler enthält. Die Estradiolfluxraten liegen bei den TTS mit höherer Estradiolkonzentration (Beispiele 2 und 3) ebenfalls über den für das Handelspräparat gemessenen Werten.

**Tabelle 2**

| (1) Levonorgestrel (LN)-gehalt und -fluxraten durch exzidierte Mäusehaut | | | |
|---|---|---|---|
| TTS-Prüfpräparat | Gehalt LN Gew.-% bezogen auf die Polymermatrix | Kumulative LN Fluxraten (µg/cm²), Mittelwerte, n=3 nach 24 h | nach 48 h |
| Beispiel 1 | 2,69 | 13,47 | 27,40 |
| Beispiel 2 | 2,44 | 16,89 | 31,11 |
| Beispiel 3 | 2,47 | 13,02 | 25,43 |
| Beispiel 4 | 1,70 | 6,50 | --- |
| Handelspräparat (Matrixpflaster) | 0,84 | 6,15 | 11,48 |

| (2) Estradiol (E2)- gehalt und -fluxraten durch exzidierte Mäusehaut | | | |
|---|---|---|---|
| TTS-Prüfpräparat | Gehalt E2 Gew.-% bezogen auf die Polymermatrix | Kumulative E2 Fluxraten Fluxraten (µg/cm²), Mittelwerte, n=3 nach 24 h | nach 48 h |
| Beispiel 1 | 1,85 | 7,77 | 15,92 |
| Beispiel 2 | 4,94 | 28,57 | 54,14 |
| Beispiel 3 | 4,98 | 21,49 | 42,56 |
| Handelspräparat (Matrixpflaster) | 2,23 | 16,70 | 31,64 |
| Erläuterungen: --- = nicht bestimmt | | | |

## Patentansprüche

1. Transdermales Therapeutisches System (TTS) zur transepidermalen Verabreichung von Sexualsteroiden über längere Zeiträume, **dadurch gekennzeichnet, daß** das TTS eine steroidhormonhaltige Matrixmasse in Form einer Schicht aufweist, die mindestens 2 Gewichtsprozent Levonorgestrel oder mindestens 2 Gewichtsprozent Levonorgestrel und mindestens 2 Gewichtsprozent Estradiol, ammoniogruppenhaltige (Meth)acrylatcopolymere, mindestens einen Weichmacher aus der Gruppe der Ester schwacher organischer Säuren, ein Fettsäureester und Polyethylenglykol enthält.

2. TTS nach Anspruch 1, **dadurch gekennzeichnet, daß** es mit Ausnahme seiner Freisetzungsfläche auf der Haut von einem größeren wirkstofffreien Hautpflaster zur Fixierung an der Applikationsstelle umgeben ist.

3. TTS nach Anspruch 1, **dadurch gekennzeichnet, daß** es hautseitig einen wirkstofffreien Klebefilm, bestehend aus vemetztem Acrylat-Copolymeren, zur Fixierung an der Applikationsstelle enthält.

4. TTS nach Ansprüchen 1 bis 3, **dadurch gekennzeichnet, daß** die steroidhormonhaltige Matrixmasse eine feste Lösung ist.

5. TTS nach Ansprüchen 1 bis 4, **dadurch gekennzeichnet, daß** die steroidhormonhaltige Matrix als Weichmacher Zitronensäuretriester enthält.

6. TTS nach Ansprüchen 1 bis 5, **dadurch gekennzeichnet, daß** die steroidhormonhaltige Matrix als Fettsäureester einen Ester der Ölsäure oder Nonansäure enthält.

7. TTS nach Ansprüchen 1 bis 6, **dadurch gekennzeichnet, daß** die Trägerfolie matrixseitig eine Metalldampf- oder Oxidbeschichtung aufweist.

8. Verfahren zur Herstellung eines TTS nach Ansprüchen 1, 4, 5 und 6, **dadurch gekennzeichnet, daß**
(1) eine homogene beschichtungsfähige steroidhormonhaltige Matrixmasse durch Schmelzextrusion erzeugt wird, indem in einer bis zu 200°C heißen Polymerschmelze, bestehend aus einem ammoniogruppenhaltigen (Meth)acrylatcopolymeren, mindestens einem Weichmacher und Polyethylenglykol in Mischung mit einem Fettsäureester, mindestens 2 Gewichtsprozent Levonorgestrel oder mindestens 2 Gewichtsprozent Levonorgestrel und mindestens 2 Gewichtsprozent Estradiol kontinuierlich eingewogen und ungeschmolzen eingearbeitet werden,
(2) kontinuierlich ein Träger mit der nach (1) erzeugten heißen wirkstoffhaltigen Polymerschmelze in einer Dicke von 0,02 bis 0,4 mm beschichtet wird,
(3) das gemäß (2) erhaltene 2-Schichttaminat mit einer Deckschicht versehen wird.

9. Verfahren zur Herstellung eines TTS nach Ansprüchen 8 und 2, **dadurch gekennzeichnet, daß** auf das verfahrensgemäß erhaltene mit einer Deckschicht versehene 2-Schichtlaminat ein größeres wirkstofffreies Pflaster zur Fixierung des TTS auf der Haut aufgebracht wird.

10. Verfahren zur Herstellung eines TTS nach Ansprüchen 8 und 3, **dadurch gekennzeichnet, daß** hautseitig ein wirkstofffreier Klebefilm, bestehend aus vernetztem Acrylat-Copolymeren, zur Fixierung an der Haut aufgebracht wird.

11. Verfahren zur Herstellung eines TTS nach Anspruch 1, **dadurch gekennzeichnet, daß** entsprechend Anspruch 8 Levonorgestrel allein mit mindestens 2 Gewichtsprozent kontinuierlich eingewogen und ungeschmolzen eingearbeitet wird.

## Claims

1. Transdermal therapeutic system (TTS) for transepidermal administering of sexual steroids over extended temporal periods, **characterised in that** the TTS comprises a steroid hormone containing matrix mass in the form of a layer which contains at least 2 weight-% Levonorgestrel or at least 2 weight-% Levonorgestrel and at least 2 weight-% Estradiol, ammonio-group containing (meth)acrylate copolymers, at least one plasticiser of the group of ester weak organic acids, a fatty acid ester and polyethylene glykol.

2. TTS according to Claim 1, **characterised in that** it is, with the exception on its release surface on the skin, surrounded by a larger non-active skin plaster for fixing to the point of application.

3. TTS according to Claim 1, **characterised in that** it comprises at the side facing the skin a non-active adhesive film of bonded acrylate copolymers for fixing to the point of application.

4. ITS according to Claims 1 to 3, **characterised in that** the steroid hormone containing matrix mass is a solid solution.

5. TTS according to Claims 1 to 4, **characterised in that** the steroid hormone containing matrix contains a citric acid triester as plasticiser.

6. TTS according to Claims 1 to 5, **characterised in that** the steroid hormone containing matrix contains an ester of oleic acid or nonane acid as fatty acid ester.

7. TTS according to Claims 1 to 6, **characterised in that** the support foil is at the side of the matrix provided with a metal vapour or oxide coating.

8. Method of producing a TTS according to Claims 1, 4, 5 and 6, **characterised in that**
(1) a homogenous coatable steroid hormone containing matrix mass is produced by way of melt extrusion **in that** the polymer melt, which is heated up to 200°C and contains an ammonio-group containing (meth)acrylate copolymer, at least one plasticiser and polyethylene glykol mixed with a fatty acid ester, at least 2 weight-% Levonogetrel or at least 2 weight-% Levonorgestrel and at least 2 weight-% Estradiol continuously weighed in and incorporated unmelted;
(2) a support is continuously coated at a thickness between 0.02 and 0.4 mm with an active component containing polymer melt produced according to (1);
(3) the 2-layer laminate obtained according to (2) is provided with a covering layer.

9. Method of producing a TTS according to Claims 8 and 2, **characterised in that** onto the 2-layer laminate obtained according to the method and provided with a covering layer is applied a larger non-active plaster for fixing the TTS onto the skin.

10. Method of producing a TTS according to Claims 8 and 3, **characterised in that** a non-active adhesive film at the side of the skin composed of bonded acrylate copolymers is applied for fixing to the skin.

11. Method of producing a TTS according to Claim 1, **characterised in that** according to Claim 8 Levonorgestrel is incorporated on its own with at least 2 weight-% continuously weighed in unmelted.

## Revendications

1. Système thérapeutique transdermique (STT) servant à l'administration transdermique de stéroïdes sexuels sur des périodes prolongées, ***caractérisé en ce que*** le STT présente une masse matricielle contenant des hormones stéroïdiennes sous la forme d'une couche qui contient au moins 2 % en poids de levonorgestrel ou au moins 2 % en poids de levonorgestrel et au moins 2 % en poids d'estradiol, des copolymères de (méth)acrylate contenant des groupes ammonio, au moins un plastifiant du groupe des esters d'acides organiques faibles, un ester d'acide gras et du polyéthylène glycol.

2. Système thérapeutique transdermique selon la revendication 1, ***caractérisé en ce qu***'à l'exception de sa surface de libération sur la peau, il est entouré d'un pansement cutané plus grand dépourvu de principe actif pour la fixation sur la zone d'application.

3. Système thérapeutique transdermique selon la revendication 1, ***caractérisé en ce qu***'il contient côté peau un film adhésif dépourvu de principe actif, composé de copolymères d'acrylate réticulés, pour fixation sur la zone d'application.

4. Système thérapeutique transdermique selon les revendications 1 à 3, ***caractérisé en ce que*** la masse matricielle contenant des hormones stéroïdiennes est une solution solide.

5. Système thérapeutique transdermique selon les revendications 1 à 4, ***caractérisé en ce que*** la masse matricielle contenant des hormones stéroïdiennes contient du triester d'acide citrique comme plastifiant.

6. Système thérapeutique transdermique selon les revendications 1 à 5, ***caractérisé en ce que*** la masse matricielle contenant des hormones stéroïdiennes contient, comme ester d'acide gras, un ester de l'acide oléique ou de l'acide nonanoïque.

7. Système thérapeutique transdermique selon les revendications 1 à 6, ***caractérisé en ce que*** la feuille support présente côté matrice un revêtement de vapeur métallique ou d'oxyde.

8. Procédé de fabrication d'un système thérapeutique transdermique selon les revendications 1, 4, 5 et 6, ***caractérisé en ce que***
(1) une masse matricielle homogène enductible contenant des hormones stéroïdiennes est produite par extrusion de matière fondue en dosant et intégrant à l'état non fondu et de manière continue, dans une masse fondue de polymères chaude à une température allant jusqu'à 200°C et composée d'un copolymère de (méth)acrylate contenant des groupes ammonio, au moins un plastifiant et du polyéthylène glycol en mélange avec un ester d'acide gras, au moins 2 % en poids de levonorgestrel ou au moins 2 % en poids de levonorgestrel et au moins 2 % en poids d'estradiol,
(2) un support est enduit de manière continue avec la masse fondue de polymères chaude produite en (1) et contenant des principes actifs, sur une épaisseur de 0,02 à 0,4 mm,
(3) le stratifié à deux couches obtenu en (2) est muni d'une couche de couverture.

9. Procédé de fabrication d'un système thérapeutique transdermique selon les revendications 8 et 2, ***caractérisé en ce qu***'un pansement plus grand sans principe actif est appliqué sur le stratifié à deux couches muni d'une couche de couverture et obtenu selon le procédé, pour la fixation du STT sur la peau.

10. Procédé de fabrication d'un système thérapeutique transdermique selon les revendications 8 et 3, ***caractérisé en ce que*,** côté peau, un film adhésif sans principe actif, composé de copolymères d'acrylate réticulés, est appliqué pour la fixation sur la peau.

11. Procédé de fabrication d'un système thérapeutique transdermique selon la revendication 1, ***caractérisé en ce que,*** comme à la revendication 8, du levonorgestrel seul est dosé et intégré à l'état non fondu et de manière continue à raison d'au moins 2 % en poids.
